# EUROPEAN PATENT APPLICATION

(11) **EP 4 205 834 A1**
(43) Date of publication of application: **05.07.2023**
(21) Application number: 21861242.2
(22) Date of filing: 11.08.2021
(51) Int. Cl.: B01D 59/04, C07C 19/08, C07C 17/383

(54) **CARBON STABLE ISOTOPE ENRICHMENT METHOD**

(30) Priority: 26.08.2020 JP 2020142320
(71) Applicant: Taiyo Nippon Sanso Corporation, Tokyo 142-8558 (JP)
(72) Inventor: SAKURAI, Hayato, Tokyo 142-8558 (JP); KAMBE, Takashi, Tokyo 142-8558 (JP); IGARASHI, Takehiro, Tokyo 142-8558 (JP)
(74) Representative: HGF
(86) International application number: PCT/JP2021/029675
(87) International publication number: WO 2022/044814

(57) **Abstract**

An object of the present invention is to provide a carbon stable isotope enrichment method that does not require safety measures against combustibility and toxicity and that enables high-concentration ¹³C enrichment without isotope scrambling, and the present invention provides a carbon stable isotope enrichment method including a distillation step for enriching carbon tetrafluoride stable isotope molecules containing ¹³C by distilling raw material carbon tetrafluoride containing multiple types of carbon tetrafluoride stable isotope molecules in a distillation column group in which multiple distillation columns are cascaded.

## Description

### TECHNICAL FIELD

The present invention relates to a carbon stable isotope enrichment method.

### BACKGROUND ART

Carbon stable isotopes (¹³C) are currently mainly used as tracers and the like in the fields of natural science and medicine. Moreover, in recent years, the demand for ¹³C as a raw material for urea breath tests is increasing. A urea breath test is used to confirm infection with Helicobacter pylori, which is the cause of gastric cancer. When ¹³C is used as a raw material for urea breath tests, it is required that the concentration of ¹³C is as high as 99% or more.

A distillation cascade process is known as a separation and enrichment method for stable isotopes. A distillation cascade process is a process in which a plurality of distillation columns are connected in a cascade manner, and specific components enriched in each distillation column are supplied into adjacent distillation columns for further enrichment.

As a method of enriching ¹³C, a method of enriching carbon monoxide including stable isotope (¹³CO) containing ¹³C by using the distillation cascade process for carbon monoxide distillation is known (Patent Document 1).

Carbon has two types of stable isotope, ¹²C and ¹³C, and the abundance percentage in nature is 98.9% and 1.1%, respectively. On the other hand, oxygen has three types of stable isotope, ¹⁶O, ¹⁷O and ¹⁸O, and the abundance percentage in nature is 99.76 %, 0.04 %, and 0.20 %, respectively. Thus, there are six types of carbon monoxide including a stable isotope: ¹²C¹⁶O, ¹²C¹⁷O, ¹²C¹⁸O, ¹³C¹⁶O, ¹³C¹⁷O and ¹³C¹⁸O.

Carbon monoxide distillation employs a method of enriching ¹³C¹⁶O and ¹³C¹⁸O among molecules containing stable isotope ¹³C in a distillation column at the end of a distillation cascade process.

However, in the carbon monoxide distillation, since ¹²C¹⁸O is also enriched at the same time, ¹³C enrichment is inhibited, and ¹³C¹⁶O and ¹³C¹⁸O could not be enriched to a high concentration of 99% or more in total.

In order to solve the problems above, a technique combining isotope scrambling in addition to the distillation cascade process has been used (Non-Patent Document 1). Isotopic scrambling is an exchange reaction that randomly rearranges the atoms constituting a compound.

In this technique, a part of the ¹²C¹⁸O enriched in the distillation cascade process is first converted to ¹³C¹⁸O by isotope scrambling (¹³C¹⁶O + ¹²C¹⁸O → ¹²C¹⁶O + ¹³C¹⁸O), to obtain enriched products improved in the concentration of ¹³C¹⁸O. The enrichment is then returned into the distillation column and further enriched in a distillation cascade process to enrich ¹³C¹⁶O and ¹³C¹⁸O in higher yields. By this technique, carbon monoxide enriched with a total of 99% or more of ¹³C¹⁶O and ¹³C¹⁸O can be obtained.

### PRIOR ART DOCUMENTS

### PATENT DOCUMENTS

Patent Document 1: Japanese Unexamined Patent Application, First Publication No. 2018-164884

### NON-PATENT DOCUMENTS

Non-Patent Document 1: B. B. McInteer, "Separation Science and Technology", 15th Edition, (3), 1980, p 491-508

### SUMMARY OF INVENTION

### PROBLEM TO BE SOLVED BY THE INVENTION

As described above, in carbon monoxide distillation, isotope scrambling is an essential process for enrichment of ¹³C to a high concentration of 99% or more. As a result, the process becomes complicated, and equipment costs such as devices for performing isotope scrambling (isotope scrambler, isotope-enriched gas lead-out line, isotope-enriched gas return line) are required.

In addition, carbon monoxide is combustible and toxic. Therefore, when installing a plant for carbon monoxide distillation, safety measures such as the use of explosion-proof equipment, the installation of an external vacuum chamber for preventing gas leakage, and the installation of alarms are costly.

The present invention has been made in view of the above problems, and an object of the present invention is to provide a carbon stable isotope enrichment method that does not require safety measures against combustibility and toxicity and that enables high-concentration ¹³C enrichment without isotope scrambling.

### MEANS FOR SOLVING THE PROBLEM

In order to achieve the object above, the present invention employs the following configurations.
[1] A carbon stable isotope enrichment method including a distillation step of distilling raw material carbon tetrafluoride containing multiple types of carbon tetrafluoride containing a stable isotope in a distillation column group in which multiple distillation columns are cascaded, and enriching carbon tetrafluoride containing a stable isotope ¹³C.

### EFFECTS OF THE INVENTION

According to the carbon stable isotope enrichment method of the present invention, carbon stable isotope can be enriched to a high concentration without requiring safety measures against flammability and toxicity and without performing isotope scrambling.

### BRIEF DESCRIPTION OF DRAWINGS

[FIG. 1] FIG. 1 is a system diagram showing a main part of an enrichment apparatus for a carbon stable isotope applicable to a carbon stable isotope enrichment method according to an embodiment of the present invention.
[FIG. 2] FIG. 2 is a system diagram showing a main part of an enrichment apparatus for carbon tetrafluoride containing a stable isotope used in Example 1.
[FIG. 3] FIG. 3 is a system diagram showing a main part of an enrichment apparatus for carbon monoxide containing a stable isotope used in Comparative Example 1.

### DETAILED DESCRIPTION OF THE PREFERRED EMBODIMENTS

### EXPLANATION OF REFERENCE NUMERALS

Hereinafter, the present invention will be described in detail by showing embodiments with reference to the accompanying drawings.

In addition, in figures used in the following explanation, in order to make the features easier to understand, the characteristic portions may be enlarged for convenience, and the dimensional ratios of each component may not necessarily be the same as the actual ones.

### <Carbon stable isotope enrichment apparatus>

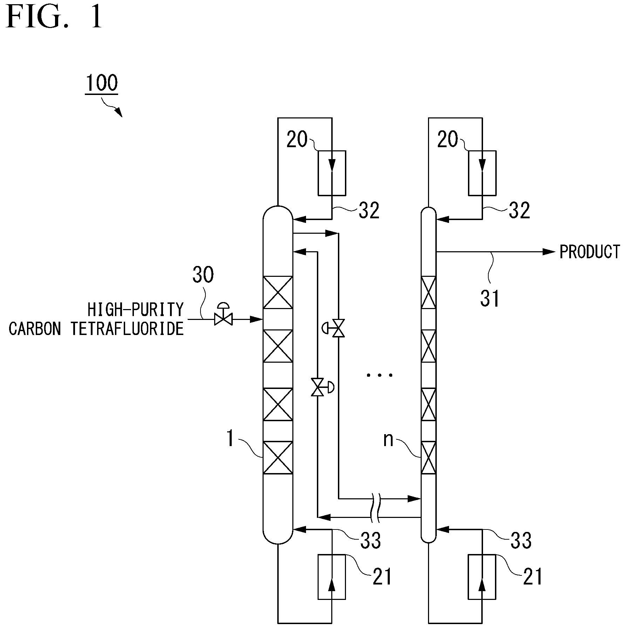

FIG. 1 is a system diagram showing a main part of an enrichment apparatus for a carbon stable isotope applicable to the carbon stable isotope enrichment method according to the embodiment of the present invention.

The carbon stable isotope enrichment apparatus 100 of the present embodiment includes a distillation column group in which a plurality of distillation columns are cascaded, a plurality of condensers 20, a plurality of reboilers 21, a raw material feed line 30, and a product line 31.

Hereinafter, an n-th distillation column from the upstream end of the group of distillation columns will be referred to as the n-th distillation column.

In FIG. 1, due to space limitations, among the first to n-th distillation columns 1 to n, the first distillation column 1 to which the raw material high-purity carbon tetrafluoride is supplied, and the n-th distillation column are only shown.

The first to n-th distillation columns 1 to n are cascade-connected in order of column number.

The first to n-th distillation columns 1 to n distill cooled carbon tetrafluoride at cryogenic temperatures. As a result, carbon tetrafluoride containing a carbon stable isotope with high vapor pressure are enriched on the column top side, and carbon tetrafluoride containing a carbon stable isotope with low vapor pressure are enriched on the column bottom side.

Among the first to n-th distillation columns 1 to n, the first distillation column 1 to which the raw material is supplied has the largest distillation load and therefore has the largest column diameter. The distillation load gradually decreases from the first distillation column 1 toward the end (column diameter decreases).

One condenser 20 is provided with each distillation column (first to n-th distillation columns 1 to n).

The condenser 20 is provided with a circulation line 32 of which ends are connected to different positions on the top of each distillation column. The condenser 20 has a function of liquefying gas that has arisen in the distillation column by exchanging heat and causing it to descend again in the distillation column.

One reboiler 21 is provided with each distillation column.

The reboiler 21 is provided in a circulation line 33 of which ends are connected to different positions on the bottom of each distillation column. The reboiler 21 has a function of vaporizing liquid that has descended in the distillation column by exchanging heat and causing it to ascend again in the distillation column.

One end of the raw material feed line 30 is connected to the middle portion of the first distillation column 1. The raw material feed line 30 is a line for supplying high-purity carbon tetrafluoride (raw material carbon tetrafluoride) to the middle portion of the first distillation column 1. The raw material feed line 30 is provided with a valve.

The middle portion of the distillation column refers to positions other than the top and bottom of the distillation column.

"High purity" means that the purity of carbon tetrafluoride is 99.999% or higher.

The high-purity carbon tetrafluoride supplied from the raw material feed line 30 contains two types of molecules containing a stable isotope shown in Table 1 below. The abundance percentage of each molecule containing as stable isotope is generally similar to the natural abundance percentage shown in Table 1.

**[Table 1]**

| Molecule containing stable isotope | Molecular weight | Natural abundance percentage [%] |
|---|---|---|
| ¹²CF₄ | 88 | 98.9 |
| ¹³CF₄ | 89 | 1.1 |

The product line 31 is a line for leading out enriched ¹³CF₄ as a product from the n-th distillation column n. One end of the product line 31 is connected to the n-th distillation column n. The ¹³CF₄ product is carbon tetrafluoride containing highly enriched (for example, 99% or greater) ¹³CF₄.

Carbon tetrafluoride is distilled in the carbon stable isotope enrichment apparatus 100 described above. Since carbon tetrafluoride has two kinds of molecules containing a stable isotope (¹²CF₄ and ¹³CF₄), ¹³CF₄ with a high concentration of 99% or more can be obtained without isotope scrambling. As a result, equipment costs such as an isotope scrambler are not required, and the carbon stable isotope can be enriched to a high concentration of 99% or more in a small number of steps.

In addition, carbon tetrafluoride is inert and non-toxic, so safety measures are not required when installing a plant that performs carbon tetrafluoride distillation. Therefore, equipment costs can be greatly reduced.

Furthermore, the resulting ¹³CF₄ product is useful as a tracer in the medical field.

### <Carbon stable isotope enrichment method>

Next, a carbon stable isotope enrichment method according to an embodiment of the present invention will be described with reference to FIG. 1. In this embodiment, the carbon stable isotope enrichment apparatus 100 shown in FIG. 1 is used to enrich ¹³CF₄.

The carbon stable isotope enrichment method of this embodiment includes a distillation step.

In the distillation step, high-purity carbon tetrafluoride (a raw material carbon tetrafluoride containing a plurality of types of carbon tetrafluoride containing a stable isotope) is supplied into the first distillation column, and distilled in the distillation column group (first to n-th distillation columns 1 to n) to enrich carbon tetrafluoride containing a stable isotope ¹³C.

The carbon stable isotope enrichment method of this embodiment uses carbon tetrafluoride as a raw material containing carbon a stable isotope.

The high-purity carbon tetrafluoride supplied from the raw material feed line 30 contains two types of molecules containing a stable isotope shown in Table 1 above. Also, the abundance percentage of each molecule containing a stable isotope is generally similar to the natural abundance percentage shown in Table 1.

Immediately after starting the distillation step (immediately after operating each distillation column), the concentration of each molecule containing a stable isotope is uniform in all the distillation columns (first to n-th distillation columns 1 to n). Then, over time, ¹³CF₄ is enriched in the n-th distillation column n. Finally, ¹³CF₄ enriched to a high concentration of 99% or more is led out from the n-th distillation column n through the product line 31.

In the carbon stable isotope enrichment method described above, carbon tetrafluoride is used as a raw material containing a carbon stable isotope and distilled. Since carbon tetrafluoride has two kinds of molecules containing a stable isotope (¹²CF₄ and ¹³CF₄), ¹³CF₄ with a high concentration of 99% or more can be obtained without isotope scrambling. As a result, since equipment such as an isotope scrambler is unnecessary, equipment costs can be reduced. In addition, carbon stable isotope can be enriched to a high concentration of 99% or more in a small number of steps.

In addition, since carbon tetrafluoride is inert and non-toxic, no safety precautions are required when setting up a plant for carbon tetrafluoride distillation. Therefore, the cost can be greatly reduced.

In addition, the resulting ¹³CF₄ product is useful as a tracer in the medical field.

Although one example of the carbon stable isotope enrichment method and the carbon stable isotope enrichment apparatus of the present invention has been described above by showing embodiments, the present invention is not limited to these embodiments. Each configuration and combination thereof in the embodiments above are examples, and addition, omission, replacement, and other modifications of the configuration are possible without departing from the scope of the present invention.

For example, the distillation column may be a packed column packed with regular packings, a packed column packed with random packings, or a plate column.

The condenser 20 and reboiler 21 above are not essential components for each distillation column, and may not be provided if the gas ascends sufficiently due to the pressure difference between the front and rear distillation columns, the pump, the blower, and the like.

### EXAMPLES

The present invention will be described in detail below with reference to Examples, but the present invention is not limited thereto.

### (Example 1)

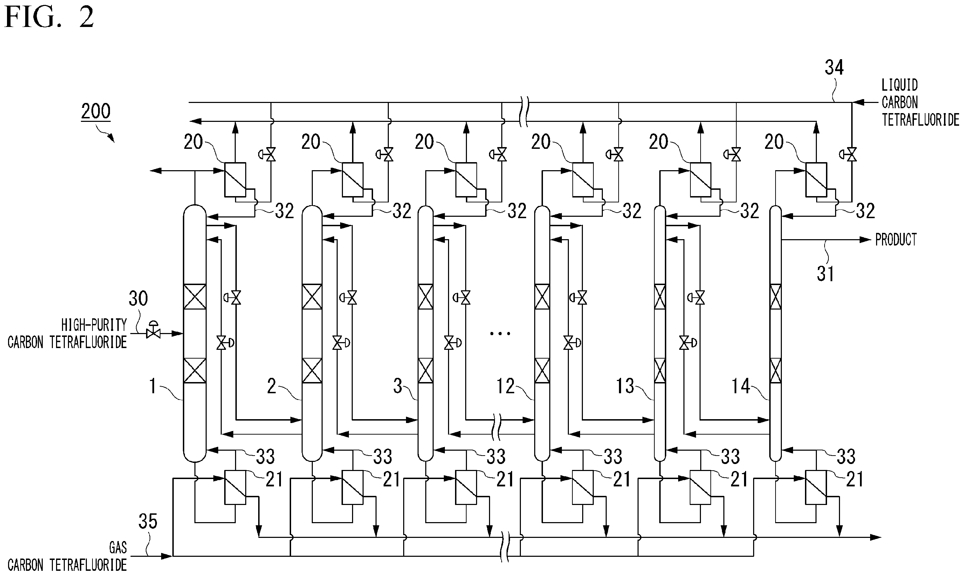

In Example 1, enrichment of ¹³CF₄ was performed using the enrichment apparatus 200 for carbon tetrafluoride containing a stable isotope shown in FIG. 2.

The basic configuration of the enrichment apparatus 200 for carbon tetrafluoride containing a stable isotope is the same as the carbon stable isotope enrichment apparatus 100. The group of distillation columns is made of 14 distillation columns, and distillation columns 1 to 14 are packed columns packed with regular packings.

The composition of the high-purity carbon tetrafluoride supplied (raw material carbon tetrafluoride) was as shown in Table 1 above. The feed rate of high-purity carbon tetrafluoride was 20 Nm³/h.

Liquid carbon tetrafluoride was used as a cold source for the condensers 20, and the liquefied carbon tetrafluoride was supplied to each condenser 20 from the liquefied carbon tetrafluoride supply line 34. Gas carbon tetrafluoride was used as a heat source for the reboiler 21, and gas carbon tetrafluoride was supplied to each reboiler 21 from the gas carbon tetrafluoride supply line 35.

Table 2 shows the abundance percentage of the molecules containing as stable isotope in the ¹³CF₄ product led out from the 14th distillation column 14 when the apparatus is stable (during steady operation).

In addition, the power required to operate the apparatus was 480 kW.

**[Table 2]**

| Molecule containing stable isotope | Molecular weight | Abundance percentage [%] | Carbon isotope | Abundance percentage [%] |
|---|---|---|---|---|
| ¹²CF₄ | 88 | 0.3 | 12C | 0.3 |
| ¹³CF₄ | 89 | 99.7 | 13C | 99.7 |

### (Comparative Example 1)

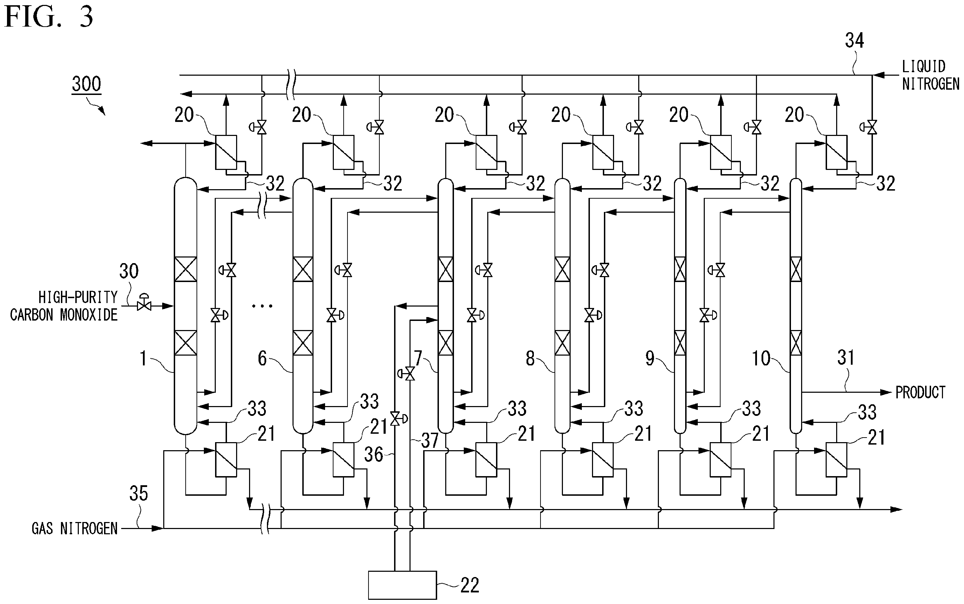

In Comparative Example 1, ¹³CO was enriched using an enrichment apparatus 300 for carbon monoxide containing a stable isotope shown in FIG. 3.

The enrichment apparatus 300 for carbon monoxide containing a stable isotope has the same structure as that of the enrichment apparatus 200 for carbon tetrafluoride containing a stable isotope, except that the enrichment apparatus 300 for carbon monoxide containing a stable isotope includes ten distillation columns, and is equipped with an isotope scrambler 22, an isotope-enriched gas lead-out line 36, and an isotope-enriched gas return line 37.

The isotope enriched-gas lead-out line 36 has one end connected to the middle portion of the seventh distillation column 7 and the other end connected to the isotope scrambler 22 to lead out some or all of the carbon monoxide, and supply into the isotope scrambler 22. In the isotope scrambler 22, the isotope exchange reaction between ¹³C¹⁶O and ¹²C¹⁸O (¹³C¹⁶O + ¹²C¹⁸O → ¹²C¹⁶O + ¹³C¹⁸O) yields an enriched-product with a higher concentration of ¹³C¹⁸O. The isotope enriched-gas return line 37 returns the enriched-product in which the concentration of ¹³C¹⁸O is further increased by the isotope exchange reaction to the seventh distillation column 7.

The composition of the high-purity carbon monoxide supplied (raw material carbon monoxide) was as shown in Table 3. The feed rate of high-purity carbon monoxide was 20 Nm³/h.

Liquid nitrogen was used as a cold source for the condensers 20, and the liquefied nitrogen was supplied into each condenser 20 from the liquefied nitrogen supply line 34. Gas nitrogen was used as the heat source for the reboiler 21, and the gas nitrogen was supplied into each reboiler 21 from the gas nitrogen supply line 35.

Simultaneously with the operation of the apparatus, leading out of carbon monoxide from the seventh distillation column 7, isotope scrambling in the isotope scrambler 22, and return of the enriched-product from the isotope scrambler 22 into the seventh distillation column 7 are performed.

**[Table 3]**

| Stable isotope molecule | Molecular weight | Natural abundance percentage [%] |
|---|---|---|
| ¹²C¹⁶O | 28 | 98.6617 |
| ¹²C¹⁷O | 29 | 0.0366 |
| ¹³C¹⁶O | 29 | 1.0973 |
| ¹³C¹⁷O | 30 | 0.0004 |
| ¹²C¹⁸O | 30 | 0.2018 |
| ¹³C¹⁸O | 31 | 0.0022 |

Table 4 shows the abundance percentage of the molecules containing a stable isotope in the ¹³CO product led out from the tenth distillation column 10 when the apparatus is stable (during steady operation).

In addition, the power required to operate the apparatus was 450 kW.

**[Table 4]**

| Molecule containing stable isotope | Molecular weight | Abundance percentage[%] |
|---|---|---|
| ¹²C¹⁶O | 28 | 0.04 |
| ¹²C¹⁷O | 29 | 0 |
| ¹³C¹⁶O | 29 | 97.11 |
| ¹³C¹⁷O | 30 | 0.15 |
| ¹²C¹⁸O | 30 | 0.26 |
| ¹³C¹⁸O | 31 | 2.44 |
| Stable isotope | Atomic weight | Abundance percentage[%] |
| 12C | 12 | 0.3 |
| 13C | 13 | 99.7 |

In Example 1, ¹³C could be enriched to a high concentration of 99% or more without isotope scrambling. In addition, in Example 1, since ¹³CF₄, which is inert and non-toxic, was used, no safety measures were required. Therefore, in Example 1, compared with Comparative Example 1, the equipment cost could be greatly reduced.

Furthermore, in Example 1, the power required to operate the apparatus was almost the same as in Comparative Example 1, so the carbon stable isotope could be enriched to a high concentration of 99% or more at a lower cost than in Comparative Example 1.

### INDUSTRIAL APPLICABILITY

The present invention can be used as a carbon stable isotope enrichment method for enriching carbon tetrafluoride (¹³CF₄) containing a stable isotope, which exist very rarely in nature by using a distillation apparatus for distilling a cryogenic fluid, wherein the distillation apparatus includes a group of distillation columns in which a plurality of distillation columns are cascaded.

### EXPLANATION OF REFERENCE NUMBERALS

- 1 to n: distillation column
- 20: condenser
- 21: reboiler
- 22: isotope scrambler
- 30: raw material feed line
- 31: product line
- 32: circulation line
- 33: circulation line
- 34: supply line (liquefied carbon tetrafluoride supply line, liquefied nitrogen supply line)
- 35: supply line (gas carbon tetrafluoride supply line, gas nitrogen supply line)
- 36: isotope enriched-gas lead-out line
- 37: isotope enriched-gas return line
- 100: carbon stable isotope enrichment apparatus
- 200: enrichment apparatus for carbon tetrafluoride containing a stable isotope apparatus
- 300: carbon monoxide stable isotope enrichment apparatus

## Claims

1. A carbon stable isotope enrichment method including a distillation step of distilling raw material carbon tetrafluoride containing multiple types of carbon tetrafluoride containing a stable isotope in a distillation column group in which multiple distillation columns are cascaded, and enriching carbon tetrafluoride containing a stable isotope ¹³C.
